# EUROPEAN PATENT SPECIFICATION

(11) **EP 1 791 514 B1**
(45) Date of publication and mention of the grant of the patent: **14.04.2010**
(21) Application number: 05786122.1
(22) Date of filing: 23.09.2005
(51) Int. Cl.: A61Q 19/00, A61K 8/25, A61K 8/81, A61K 8/92

(54) **EXFOLIATING AND SOFTENING COMPOSITION**
ABSCHÄLENDE UND ERWEICHENDE ZUSAMMENSETZUNG
COMPOSITION EXFOLIANTE ET ADOUCISSANTE

(30) Priority: 24.09.2004 GB 0421286; 05.07.2005 GB 0513704
(43) Date of publication of application: 06.06.2007
(73) Proprietor: Gelicity (UK) Limited, Aber Industrial Estate Flint Flintshire CH6 5YL (GB)
(72) Inventor: MORRIS, Paul, Buckley, Flintshire CH7 2DB (GB); WALTON, Wayne, Winsford, Cheshire CW7 2SY (GB)
(74) Representative: Wombwell, Francis
(86) International application number: PCT/GB2005/003674
(87) International publication number: WO 2006/032906

(56) References cited:
- EP-A- 0 361 106
- EP-A- 0 867 422
- EP-A- 1 186 286
- DE-A1- 10 259 016
- DE-A1- 10 309 179
- US-B1- 6 280 765
- PATENT ABSTRACTS OF JAPAN vol. 2003, no. 07, 3 July 2003 (2003-07-03) & JP 2003 081811 A (TOU-FREE INTERNATIONAL:KK; PURIMAARU KK), 19 March 2003 (2003-03-19)

## Description

The present invention relates to compositions that are useful for exfoliating and softening skin. In particular, the present invention relates to compositions that are useful for exfoliating and softening skin containing sodium polyacrylate powder as an active ingredient or agent dispersed in a solution of base oil.

Skin exfoliating products are well known and serve to remove dead skin cells from the surface of the skin. Generally, such exfoliating products comprise abrasive particles dispersed in a cleansing solution, such as body washes and liquid hand soaps. These abrasive particles may be natural, such as walnut shells or grit or sand, or synthetic particles, such as polyethylene beads. After application of such exfoliating products, a separate skin moisturiser is often required to replace some of the natural oils that are lost from the surface of the skin during exfoliation.

The problem associated with many commercially available skin exfoliating products is that they can be sometimes too harsh for the skin; stripping the skin of its essential natural oils and requiring the separate application of a moisturiser.

Some examples of sodium polyacrylate dispersed in a oil are described in JP 2003 081811 A, EP 0 361 106, US 6,280,765, and EP 0 867 422.

It is the object of the present invention to provide compositions that are useful for exfoliating and softening skin and which are particularly suitable for cosmetic, toiletry, spa treatments and beauty products. In use, a small quantity of the composition is applied to the skin to produce an exfoliating and smoothing effect. When the skin is then rinsed with water, the sodium polyacrylate powder swells and turns to a soft gel which allows the base oil to be released into the skin which softens and moisturises the skin. The base oil and other additives soften and moisturise even the roughest of skin types and produces a soft exfoliating feeling on the skin. The present invention therefore provides compositions that both exfoliate and soften the skin in a single treatment. In a further object of the present invention, the composition opens and has a cleansing effect on skin pores.

The present invention concerns the use of a composition comprising sodium polyacrylate powder at between 5% to 70% by weight dispersed in a solution of base oil as a product for exfoliating and softening the skin.

Preferably, said base oil comprises any suitable food grade oils, synthetic, vegetable or mineral oils. Further preferably, said base oil is selected from a group consisting of avocado oil, wheatgerm oil and grapeseed oil.

Likewise according to the present invention there is provided a skin exfoliating and softening composition, comprising sodium polyacrylate powder at between 5% to 70% by weight dispersed in a solution of base oil, said base oil being selected from a group consisting of wheatgerm oil and grapeseed oil.

Preferably, said skin exfoliating and softening composition comprises sodium polyacrylate powder at between 20% to 55% by weight. In a preferred embodiment, said skin exfoliating and softening composition comprises sodium polyacrylate powder at between 35% to 40% by weight.

Preferably, said composition may further comprise at least one additive selected from: perfumes, colours, anti-settling agents, preservatives, vitamins and skin conditioning agents. Further preferably, said anti-settling agent comprises fumed silica.

Also according to the present invention there is provided a method of exfoliating and softening skin, comprising the steps of:
applying a composition comprising sodium polyacrylate powder at between 5% to 70% by weight dispersed in a solution of base oil to the skin;
rubbing said composition into the skin for a period of time to produce an exfoliating effect; and
applying water to the skin and rubbing until all of said composition is either washed away or absorbed into the skin.

It will be appreciated that the step of applying water to the skin causes said sodium polyacrylate powder to swell and releases said base oil into the surface of the skin which moisturises the surface of the skin.

Furthermore, the present invention also provides a method of manufacturing a skin exfoliating and softening composition, comprising the steps of:
combining a blend of base oils at around 28% to 93% by weight;
gradually adding an anti-settling agent at around 2% by weight under high shear; and
adding sodium polyacrylate powder at between 5% to 70% by weight to said mixture under moderate stirring.

Preferably, the method of manufacturing a skin exfoliating and softening composition further comprises the step of adding colourants at between 0.9% to 1.3% by weight under moderate stirring.

It is believed that compositions that are useful for exfoliating and softening skin in accordance with the present invention at least address the problems outlined above. The advantages of the present invention are that compositions that are useful for exfoliating and softening skin are provided which are particularly suitable as cosmetic, toiletry, spa treatments and beauty products. Advantageously, in use, a small quantity of the composition is applied to the skin to produce an exfoliating and smoothing effect. When the skin is then rinsed with water, the sodium polyacrylate powder swells and turns to a soft gel which allows the base oil to be released into the skin which softens and moisturises the skin. Advantageously, the base oil and other additives soften and moisturise even the roughest of skin types and produces a soft exfoliating feeling on the skin. The present invention therefore provides compositions that both exfoliate and soften the skin in a single treatment. In a further advantageous aspect of the present invention, the composition opens and has a cleansing effect on skin pores.

A specific non-limiting embodiment of the invention will now be described by way of example and with reference to the accompanying drawings, in which:
Figure 1 shows a perspective view of the product of the present invention prior to use;
Figure 2 illustrates how using a stirrer the product may be agitated until a free-flowing homogenous solution is formed;
Figure 3 shows the product having a uniform consistency.
Figure 4 illustrates how the composition can be applied into the palm of the hands of the user.
Figure 5 shows use of the composition on the hands.
Figure 6 illustrates the final stage of the treatment in which the hands are rinsed in water.

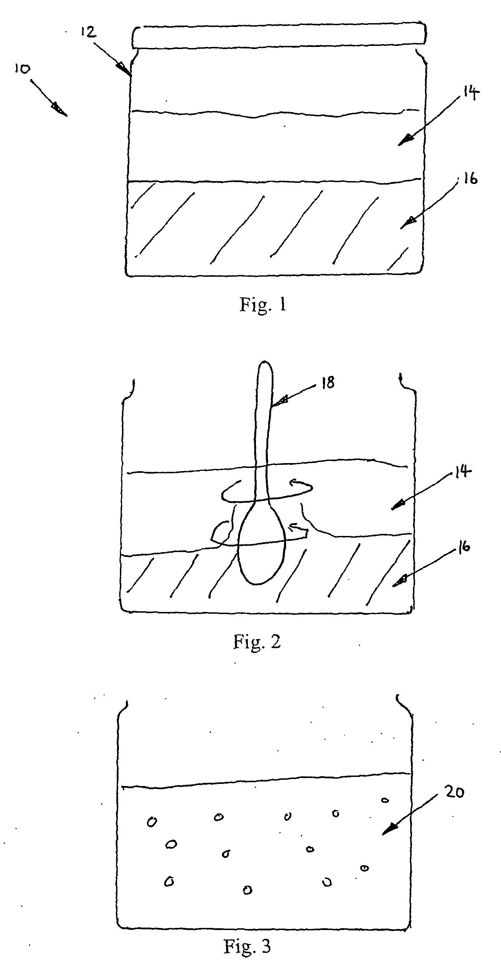

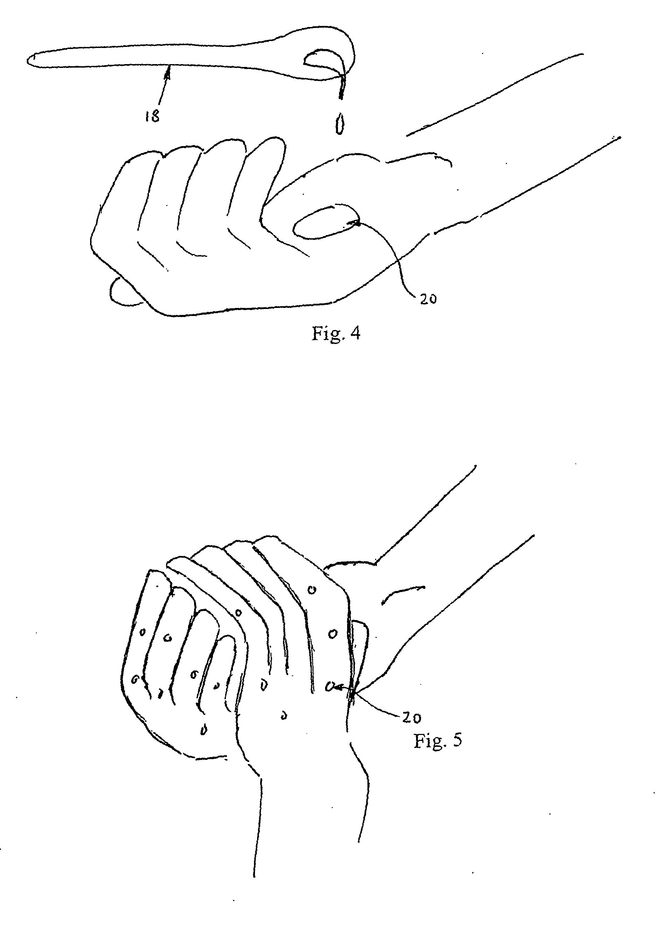

Referring now to the drawings, the use of the skin exfoliating and softening composition according to the present invention is shown in Figures 1 to 6. In particular, as shown in Figure 1, the product 10 is sold in packaging 12. Even though the composition includes anti-settling agents, the product tends to separate out over time into a base oil phase 14 and sodium polyacrylate powder 16. Figures 2 and 3 show how a stirrer, such as a spoon 18 or another suitable instrument, can be used to obtain a free-flowing viscous composition 20 having a uniform consistency. As shown in Figure 4, a small quantity of the composition 20 is placed into the palm of the hand using the spoon 18 or other measuring implement.

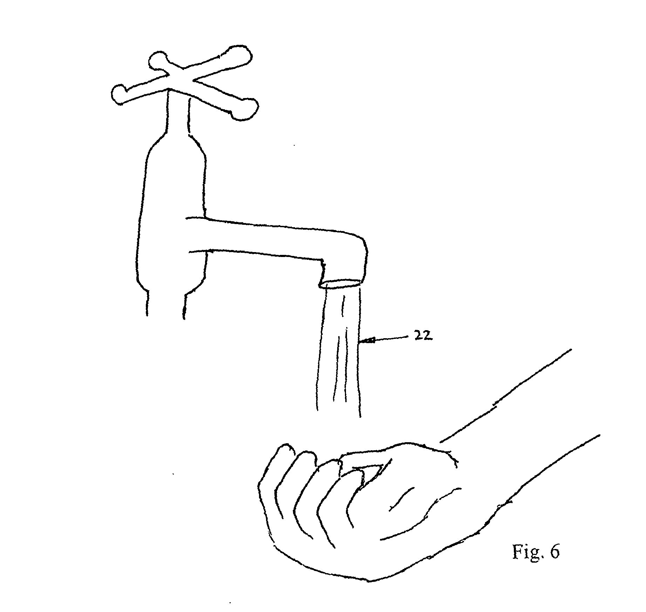

Although Figure 5 depicts the use of the product 20 for exfoliating and cleansing the hands and nails, it can of course be used on any part of the body by rubbing it over the skin to produce an exfoliating and softening effect. After rubbing the skin for a short period of time, preferably two minutes, the treated area is then placed under running water 22, as shown in Figure 6, or sprayed using an appropriate atomiser (not shown). The sodium polyacrylate powder 16 then swells and releases the base oil 14 into the skin which now moisturises the surface of the skin. At this point, the user can simply keep rubbing until all of the sodium polyacrylate powder 16 and base oil 14 is either washed away or absorbed into the skin. In use, the product 20 has exfoliating properties by firstly removing dead skin cells from the surface of the skin and deep cleansing properties by causing the skin pores to open and sucking out moisture from the skin taking with it dirt. This then allows the base oil 14 and cleaner moisture to be absorbed back into the skin leaving it feeling clean, smooth and soft. After this stage, the treated area is dried as normal.

The chemical composition of the invention will now be described. As shown in Tables 1 to 5, the present invention can be implemented using various base oils and other constituents. However, the invention should not be considered as being limited to the details thereof.

The composition shown in Table 1 is a fragrance-free product in which the sodium polyacrylate powder it utilised at 37.5% by weight dispersed in a solution of base oil, comprising a blend of avocado oil, wheatgerm oil and grapeseed oil. Watersorb PIFL 2024 is a brand name for a cosmetic grade sodium polyacrylate powder, although other generally available commercial products could of course be utilised. Aerosil®972 is a trade name for a fumed silica which is used as an anti-settling agent or dispersant for the sodium polyacrylate powder particles in the base oil phase.

**Table 1**

| Phase | Ingredient | % w/w |
|---|---|---|
| A 1 | Ethyl Panthenol | 0.2 |
| A 2 | Tocopherol | 0.1 |
| A 3 | Avocado Oil | 1 |
| A 4 | Wheatgerm Oil | |
| A 5 | α-Bisabolol | 0.05 |
| A 6 | Grapeseed Oil | 58.15 |
| | | |
| B 7 | Aerosil®972 | 2 |
| | | |
| C 8 | Watersorb PIFL 2024 | 37.5 |

The compositions shown in Tables 1 to 5 also include the constituents panthenol, tocopherol and α-Bisabolol which are particularly beneficial to the skin, since panthenol is the alcohol form of pantothenic acid, more familiar as Vitamin B5. In a living cell, panthenol is converted to pantothenic acid, which then becomes an important part of the compound 'Coenzyme A', which is important in cellular metabolism.

Vitamin E is an essential fat-soluble vitamin that includes eight naturally occurring compounds in two classes designated as tocopherols and tocotrienols, as shown in Tables 1 to 5. Vitamin E has earned itself a reputation from spicing up your love life to banning wrinkles and old age. One of the most important functions of this vitamin is its antioxidant properties. Vitamin E is an effective chain-breaking, lipid-soluble antioxidant in biological membranes, and aids in membrane stability.

α-Bisabolol is a clear, colourless to slightly yellow liquid with a faint, floral, sweetish odour. It has been used for hundreds of years in medicinal applications and this naturally occurring ingredient is used to help accelerate the healing process of the skin, and can be used confidently on sensitive skins. It has anti-irritant and anti-inflammatory properties, and it also contains anti-bacterial, as well as anti-mycotic, properties.

Tables 2 and 3 show further variants of the fragrance-free composition of Table 1, and which further include various colourants and fragrances. The preferred embodiment set forth in any of Tables 1 to 3 are particularly suitable as an exfoliating and softening composition for the hands or feet or indeed anywhere where the skin requires dead skin cells to be removed. The treatment using these compositions being particularly useful prior to a manicure or false nails being applied, as the nails and skin are scrubbed prior to treatment leaving them clean, moisturised and free of other contaminants.

**Table 2**

| Phase | Ingredient | % w/w |
|---|---|---|
| A 1 | Ethyl Panthenol | 0.2 |
| A 2 | Tocopherol | 0.1 |
| A 3 | Avocado Oil | 1 |
| A 4 | Wheatgerm Oil | 1 |
| A 5 | α-Bisabolol | 0.05 |
| A 6 | Grapeseed Oil | 56.067 |
| A 7 | Perfume PN 774.016 Kiwi & Lime | 0.8 |
| | | |
| B 8 | Aerosil®972 | 2 |
| | | |
| C 9 | Watersorb PIFL 2024 | 37.5 |
| | | |
| D 10 | Yellow 11 (CI 47000), 0.1% solution in oil | 1.074 |
| D 11 | Green 6 (CI 61565), 0.1% solution in oil | 0.209 |

**Table 3**

| Phase | Ingredient | % w/w |
|---|---|---|
| A 1 | Ethyl Panthenol | 0.2 |
| A 2 | Tocopherol | 0.1 |
| A 3 | Avocado Oil | 1 |
| A 4 | Wheatgerm Oil | 1 |
| A 5 | α-Bisabolol | 0.05 |
| A 6 | Grapeseed Oil | 56.42 |
| A 7 | Perfume PN 993.054 Mandarin & Starfruit | 0.8 |
| | | |
| B 8 | Aerosil®972 | 2 |
| | | |
| C 9 | Watersorb PIFL 2024 | 37.5 |
| | | |
| D 10 | Yellow 11 (CI 47000), 0.1% solution in oil | 0.695 |
| D 11 | Red 17 (CI 26100), 0.1% solution in oil | 0.235 |

Tables 4 and 5 show various compositions wherein the skin exfoliating and softening composition comprises sodium polyacrylate powder at between 5% and 70% by weight, respectively. Clearly, the composition shown in Table 4, which shows sodium polyacrylate powder used at 5% by weight will have a substantially reduced exfoliating effect than, say, the composition used at 37.5% or 70% by weight and finds particular application as a gentle cleansing gel for very sensitive parts of the body. Conversely, the composition shown in Table 5, which discloses a composition including sodium polyacrylate powder at 70% by weight is particularly useful as an exfoliating gel for particular parts of the body where hard and dry skin is prevalent.

**Table 4**

| Phase | Ingredient | % w/w |
|---|---|---|
| A 1 | Ethyl Panthenol | 0.2 |
| A 2 | Tocopherol | 0.1 |
| A 3 | Avocado Oil | 1 |
| A 4 | Wheatgerm Oil | 1 |
| A 5 | α-Bisabolol | 0.05 |
| A 6 | Grapeseed Oil | 90.65 |
| | | |
| B 7 | Aerosil®972 | 2 |
| | | |
| C 8 | Watersorb PIFL 2024 | 5 |

**Table 5**

| Phase | Ingredient | % w/w |
|---|---|---|
| A 1 | Ethyl Panthenol | 0.2 |
| A 2 | Tocopherol | 0.1 |
| A 3 | Avocado Oil | |
| A 4 | Wheatgerm Oil | 1 |
| A 5 | α-Bisabolol | 0.05 |
| A 6 | Grapeseed Oil | 25.65 |
| | | |
| B 7 | Aerosil®972 | 2 |
| | | |
| C 8 | Watersorb PIFL 2024 | 70 |

Any of the formulations shown in Tables 1 to 5 can be manufactured using a mixing vessel with either a sweep stirrer or a powerful propeller/turbo stirrer. A method of manufacturing a skin exfoliating and softening composition according to any of the formulations disclosed in Tables 1 to 5 will now be described. In particular, in the first stage, the ingredients of phase A are firstly mixed together and then phase B is carefully added under fast agitation and such is stirred until completely dispersed. A high shear mixer may be helpful for this stage in the manufacturing process. After phases A and B have been combined, the high shear mixer is then turned off and phase C is then added with moderate stirring. For the formulations as set forth in Tables 2 and 3, the colourants of phase D are then finally added, again, under moderate stirring.

## Claims

1. Use of a composition comprising sodium polyacrylate powder at between 5% to 70% by weight dispersed in a solution of base oil as a product for exfoliating and softening the skin.

2. The use of the composition according to claim 1, wherein the composition comprises sodium polyacrylate powder at between 20% to 55% by weight.

3. The use of the composition according to claims 1 or 2, wherein the composition comprises sodium polyacrylate powder at between 35% to 40% by weight.

4. The use of the composition according to any preceding claim, wherein said base oil comprises any suitable food grade oils, synthetic, vegetable or mineral oils.

5. The use of the composition according to any preceding claim, wherein said base oil is selected from a group consisting of avocado oil, wheatgerm oil and grapeseed oil.

6. The use of the composition according to any preceding claim, wherein the composition further comprises at least one additive selected from: perfumes, colours, anti-settling agents, preservatives, vitamins and skin conditioning agents.

7. The use of the composition according to claim 6, wherein said anti-settling agent comprises fumed silica.

8. A method of exfoliating and softening skin, comprising the steps of:
applying a composition comprising sodium polyacrylate powder at between 5% to 70% by weight dispersed in a solution of base oil to the skin;
rubbing said composition into the skin for a period of time to produce an exfoliating effect; and
applying water to the skin and rubbing until all of said composition is either washed away or absorbed into the skin.

9. The method of exfoliating and softening skin according to claim 8, wherein the step of applying water to the skin causes said sodium polyacrylate powder to swell and releases said base oil into the surface of the skin which moisturises the surface of the skin.

10. A method of manufacturing a skin exfoliating and softening composition, comprising the steps of:
combining a blend of base oils at around 28% to 93% by weight; gradually adding an anti-settling agent at around 2% by weight under high shear; and
adding sodium polyacrylate powder at between 5% to 70% by weight to said mixture under moderate stirring.

11. The method of manufacturing a skin exfoliating and softening composition according to claim 10, further comprising the step of adding colourants at between 0.9% to 1.3% by weight under moderate stirring.

12. A skin exfoliating and softening composition, comprising sodium polyacrylate powder at between 5% to 70% by weight dispersed in a solution of base oil, wherein said base oil is selected from a group consisting of wheatgerm oil and grapeseed oil.

13. The skin exfoliating and softening composition according to claim 12, comprising sodium polyacrylate powder at between 20% to 55% by weight.

14. The skin exfoliating and softening composition according to claims 12 or 13, comprising sodium polyacrylate powder at between 35% to 40% by weight.

15. The skin exfoliating and softening composition according to claims 12 to 14, further comprising at least one additive selected from: perfumes, colours, anti-settling agents, preservatives, vitamins and skin conditioning agents.

16. The skin exfoliating and softening composition according to claim 15, wherein said anti-settling agent comprises fumed silica.

## Patentansprüche

1. Verwendung einer Zusammensetzung, aufweisend zwischen 5 und 70 Gew.-% Natriumpolyacrylatpulver, dispergiert in einer Basisöllösung als Produkt zum Exfolieren und Erweichen der Haut.

2. Die Verwendung der Zusammensetzung nach Anspruch 1, wobei die Zusammensetzung zwischen 20 und 55 Gew.-% Natriumpolyacrylatpulver enthält.

3. Die Verwendung der Zusammensetzung nach Anspruch 1 oder 2, wobei die Zusammensetzung zwischen 35 und 40 Gew.-% Natriumpolyacrylatpulver enthält.

4. Die Verwendung der Zusammensetzung nach einem der vorhergehenden Ansprüche, wobei das Basisöl beliebige geeignete Lebensmittelqualität-Öle, synthetische, pflanzliche oder mineralische Öle aufweist.

5. Die Verwendung der Zusammensetzung nach einem der vorhergehenden Ansprüche, wobei das Basisöl ausgewählt ist aus einer Gruppe bestehend aus Avocadoöl, Weizenkleieöl und Traubenkernöl.

6. Die Verwendung der Zusammensetzung nach einem der vorhergehenden Ansprüche, wobei die Zusammensetzung ferner zumindest ein Additiv, ausgewählt aus Riechstoffen, Farben, Antiabsetzmitteln, Konservierungsmitteln, Vitaminen und Hautkonditionierungsmitteln aufweist.

7. Die Verwendung der Zusammensetzung nach Anspruch 6, wobei das Antiabsetzmittel pyrogene Kieselsäure umfasst.

8. Ein Verfahren zum Exfolieren und Erweichen der Haut, aufweisend die Schritte:
Aufbringen einer Zusammensetzung, aufweisend zwischen 5 und 70 Gew.-% Natriumpolyacrylatpulver, dispergiert in einer Basisöllösung, auf die Haut,
Reiben der Zusammensetzung in die Haut für einen Zeitraum zum Erzeugen eines Exfolier-Effekts, und
Aufbringen von Wasser auf die Haut und Reiben bis die gesamte Zusammensetzung entweder abgewaschen oder in der Haut absorbiert ist.

9. Das Verfahren zum Exfolieren und Erweichen von Haut nach Anspruch 8, wobei der Schritt des Aufbringens von Wasser auf die Haut bewirkt, dass das Natriumpolyacrylatpulver aufquillt und das Basisöl in die Hautoberfläche freigibt, was die Hautoberfläche befeuchtet.

10. Ein Verfahren zum Herstellen einer Haut-exfolierenden und -erweichenden Zusammensetzung, aufweisend die Schritte:
Kombinieren einer Mischung von ungefähr 28 bis 93 Gew.-% Basisölen, allmähliches Zufügen von ungefähr 2 Gew.-% eines Antiabsetzmittels unter hoher Scherkraft, und
Zufügen zwischen 5 und 70 Gew.-% Natriumpolyacrylatpulver zu der Mischung bei moderatem Rühren.

11. Das Verfahren zum Herstellen einer Haut-exfolierenden und -erweichenden Zusammensetzung nach Anspruch 10, ferner aufweisend den Schritt des Hinzufügens von zwischen 0,9 und 1,3 Gew.-% Farbstoffen bei moderatem Rühren.

12. Eine Haut-exfolierende und -erweichende Zusammensetzung, aufweisend zwischen 5 und 70 Gew.-% Natriumpolyacrylatpulver dispergiert in einer Basisöllösung, wobei das Basisöl ausgewählt ist aus einer Gruppe bestehend aus Weizenkeimöl und Traubenkernöl.

13. Die Haut-exfolierende und -erweichende Zusammensetzung nach Anspruch 12, aufweisend zwischen 20 und 55 Gew.-% Natriumpolyacrylatpulver.

14. Die Haut-exfolierende und -erweichende Zusammensetzung nach Anspruch 12 oder 13, aufweisend zwischen 35 und 40 Gew.-% Natriumpolyacrylatpulver.

15. Die Haut-exfolierende und -erweichende Zusammensetzung nach einem der Ansprüche 12 bis 14, ferner aufweisend zumindest ein Additiv ausgewählt aus Parfümen, Farbstoffen, Antiabsetzmitteln, Konservierungsmitteln, Vitaminen und Hautkonditionierungsmitteln.

16. Die Haut-exfolierende und -erweichende Zusammensetzung nach Anspruch 15, wobei, das Antiabsetzmittel pyrogene Kieselsäure umfasst.

## Revendications

1. Utilisation d'une composition comprenant une poudre de polyacrylate de sodium à hauteur de 5 % à 70 % en poids dispersée dans une solution d'huile de base en tant que produit pour exfolier et adoucir la peau.

2. Utilisation de la composition selon la revendication 1, dans laquelle la composition comprend de la poudre de polyacrylate de sodium à hauteur de 20 % à 55 % en poids.

3. Utilisation de la composition selon les revendications 1 ou 2, dans laquelle la composition comprend de la poudre de polyacrylate de sodium à hauteur de 35 % à 40 % en poids.

4. Utilisation de la composition selon l'une quelconque des revendications précédentes, dans laquelle ladite huile de base comprend toutes huiles de qualité alimentaire, huiles synthétique, végétale ou minérale appropriées.

5. Utilisation de la composition selon l'une quelconque des revendications précédentes, dans laquelle ladite huile de base est choisie dans le groupe consistant en l'huile d'avocat, l'huile de germe de blé et l'huile de pépins de raisin.

6. Utilisation de la composition selon l'une quelconque des revendications précédentes, dans laquelle la composition comprend en outre au moins un additif choisi parmi les parfums, les couleurs, les agents anti-sédimentation, les conservateurs, les vitamines et les agents de conditionnement de la peau.

7. Utilisation de la composition selon la revendication 6, dans laquelle ledit agent anti-sédimentation comprend de la silice fumée.

8. Procédé d'exfoliation et d'adoucissement de la peau, comprenant les étapes consistant à :
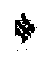 appliquer une composition comprenant une poudre de polyacrylate de sodium à hauteur de 5 % à 70 % en poids dispersée dans une solution d'huile de base à la peau ;
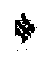 frotter ladite composition sur la peau pendant une période visant à produire un effet exfoliant ;
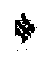 appliquer de l'eau à la peau et frotter jusqu'à ce que toute ladite composition soit éliminée par lavage ou absorbée par la peau.

9. Procédé d'exfoliation et d'adoucissement de la peau selon la revendication 8, dans lequel l'étape d'application de l'eau à la peau amène ladite poudre de polyacrylate de sodium à gonfler et libère ladite huile de base à la surface de la peau, ce qui hydrate la surface de la peau.

10. Procédé de fabrication d'une composition d'exfoliation et d'adoucissement de la peau, comprenant les étapes consistant à :
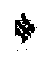 combiner un mélange d'huiles de base à environ 28 % à 93% en poids ;
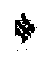 ajouter progressivement un agent anti-sédimentation à environ 2 % en poids sous un cisaillement élevé ; et
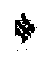 ajouter de la poudre de polyacrylate de sodium à hauteur de 5 % à 70 % en poids audit mélange sous une agitation modérée.

11. Procédé de fabrication d'une composition d'exfoliation et d'adoucissement de la peau selon la revendication 10, comprenant en outre l'étape d'ajout de colorants à hauteur de 0,9 % à 1,3 % en poids sous agitation modérée.

12. Composition d'exfoliation et d'adoucissement de la peau, comprenant de la poudre de polyacrylate de sodium à hauteur de 5 % à 70 % en poids dispersée dans une solution d'huile de base, dans laquelle l'huile de base est choisie dans le groupe consistant en l'huile de germe de blé et l'huile de pépins de raisin.

13. Composition d'exfoliation et d'adoucissement de la peau selon la revendication 12, comprenant de la poudre de polyacrylate de sodium à hauteur de 20 % à 55 % en poids.

14. Composition d'exfoliation et d'adoucissement de la peau selon les revendications 12 ou 13, comprenant de la poudre de polyacrylate de sodium à hauteur de 35 % à 40 % en poids.

15. Composition d'exfoliation et d'adoucissement de la peau selon les revendications 12 à 14, comprenant en outre au moins un additif choisi parmi : les parfums, les couleurs, les agents anti-sédimentation, les conservateurs, les vitamines et les agents de conditionnement de la peau.

16. Composition d'exfoliation et d'adoucissement de la peau selon la revendication 15, dans laquelle ledit agent anti-sédimentation comprend de la silice fumée.
